# EUROPÄISCHE PATENTSCHRIFT

(11) **EP 0 530 454 B1**
(45) Veröffentlichungstag und Bekanntmachung des Hinweises auf die Patenterteilung: **25.02.1998**
(21) Anmeldenummer: 92110437.8
(22) Anmeldetag: 20.06.1992
(51) Int. Cl.: C07C 43/205, C07C 321/28, C07C 41/16, C07C 319/14, C07C 205/37, C07C 43/215

(54) **Verfahren zur Herstellung von Alkylphenylalkylethern oder Alkylphenylalkylthioethern**
Process for the preparation of alkylphenyl alkyl ethers of alkylphenyl alkyl thioethers
Procédé pour la préparation d'éthers ou de thioéthers d'alkylphényle et d'alkyle

(30) Priorität: 13.08.1991 DE 4126671
(43) Veröffentlichungstag der Anmeldung: 10.03.1993
(73) Patentinhaber: HÜLS AKTIENGESELLSCHAFT, 45764 Marl (DE)
(72) Erfinder: Köhler, Günther, Dr., W-4370 Marl (DE); Bickert, Peter, Dr., W-4400 Münster (DE)

(56) Entgegenhaltungen:
- EP-A- 0 000 162
- EP-A- 0 104 598
- FR-A- 2 595 959
- GB-A- 2 026 484
- US-A- 4 310 706

## Beschreibung

Die Erfindung betrifft ein Verfahren zur Herstellung von Alkylphenylalkylethern durch Umsetzung von Alkylphenolen mit Dialkylcarbonat in Gegenwart von Amidin-Katalysatoren.

Alkylphenylalkylether im Sinne dieses Verfahrens sind sowohl die vom Sauerstoff abgeleiteten Ether als auch die vom Schwefel abgeleiteten Thioether.

Die Phenylether haben folgende Formel: 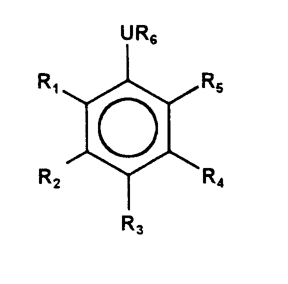
U= O,S,
R₁ bis R₆ sind gleich oder verschieden und stellen Alkyl- oder Arylgruppen dar; R₁ bis R₅ können auch andere funktionelle Gruppen sein, wie z. B. -COOR, -NO₂, -NH₂, -O-CH₂-CH₂-OH, -OH, -CHO, -Hal, sind aber nicht beschränkt darauf.

Die Reste R₁ bis R₅ können auch durch entsprechende bifunktionelle Substituenten wie z. B. -(CH₂)ₓ-, -(CH₂)ₓ-Z-(CH₂)_{y}- (Z = Hetero, x = 0-7, y = 0-7) oder insbesondere ungesättigte Substituenten überbrückt sein, wie sie für annelierte Ringsysteme, z. B. Naphtyl, Phenanthryl, Anthracenyl, Chinolyl, Isochinolyl, Indyl, aber nicht beschränkt darauf, charakteristisch sind.

Die Alkylierung von Phenolen und Thiophenolen wird üblicherweise mit Alkylestern anorganischer Säuren bzw. mit Halogencarbonsäureestern ausgeführt, wie aus Houben-Weyl "Methoden der organischen Chemie" bekannt ist. Vorteilhaft dagegen sind die Verfahren zur Herstellung von Phenylalkylethern, die in EP-PS 0 000 162 beschrieben sind. Dabei werden Phenole mit Dimethylcarbonat in Gegenwart von tert.-Aminen, Phosphinen oder der aromatischen Amine N-Methylimidazol und N-Methylpyrrol umgesetzt. Die EP-PS 0 104 598 beschreibt ebenfalls ein Alkylierungsverfahren mit Dimethylcarbonat, wobei sterisch gehinderte Alkylphenole in Gegenwart eines tert.-Amins, wie 4-(Dimethylamino)-pyridin, alkyliert werden können. Ebenfalls mit tert.-Aminen als Katalysatoren wird in EP-PS 0 315 334 die Methylierung mit Dimethylcarbonat beschrieben, wobei hier speziell ein Zuckerderivat, das Dianhydrosorbitol, methyliert wird.

Nach GB 2 026 484 werden Phenolether dadurch hergestellt, daß man Phenole mit Alkylcarbonaten in Gegenwart eines tertiären Amins und eines Jodids umsetzt. Zum Vergleich wird in Beispiel 5 die Reaktion in Gegenwart von N-Methylimidazol, einem aromatischen Diamin, genannt, wobei der Umsatz nur bei 60 % liegt.

In US 4 310 706 werden Imidazol-Katalysatoren, also ebenfalls aromatische Verbindungen, für die Alkylierung von Phenolen beansprucht.

Darüber hinaus wird in FR-PS 2 595 959 die Herstellung von Alkylphenylmethylethern durch Umsetzung von Alkylphenolen mit Dimethylcarbonat in Gegenwart von substituierten Guanidinen beschrieben.

M. Lissel et al. [Synthesis 1986, 382] setzt auch Thiophenol mit Dimethylcarbonat in Gegenwart von K₂CO₃/Kronenether um und erhielt mit ca. 80 % Ausbeute den entsprechenden Thioether.

Die Nachteile all dieser Verfahren sind, daß die Reaktionen mit Katalysatoren ausgeführt werden, die
◆ giftig sind (wie z. B. Phosphine und Kronenether),
◆ Wasserschadstoffe sind (tert.-Amine, quaternäre Ammoniumsalze, Guanidine),
◆ teuer sind (Kronenether, Guanidine),
◆ nur einmalig verwendet werden können (Guanidine),
◆ geringe Raum-Zeit-Ausbeuten gestatten (tert.-Amine),
◆ zum Teil nur unter hohem Druck Umsetzungen ermöglichen (Phosphine).

Ein besonderer Nachteil der mit Guanidinen katalysierten Veretherung besteht in der Neigung dieser Stickstoffbasen, Substituenten vom Katalysator auf das zu alkylierende Phenol unter Bildung unerwünschter Nebenprodukte sowie unter Zerstörung des katalytisch wirksamen Guanidinsystems zu übertragen. Dies bedeutet eine wesentliche Einschränkung sowohl hinsichtlich der Lebensdauer des Katalysators als auch hinsichtlich der Auswahl eines geeigneten, nicht zu unerwünschten Nebenreaktionen führenden Guanidins.

Es ist demzufolge Aufgabe der Erfindung, ein Verfahren zur Herstellung von Phenylalkylethern zu entwickeln, wobei umweltverträgliche Katalysatoren eingesetzt werden, die gut verfügbar und wiederverwendbar sind und unter geringsten Nebenreaktionen mit hoher Raum-Zeit-Ausbeute eine Synthese gestatten.

Gelöst wurde die Aufgabe dadurch, daß cyclische Amidinbasen als Katalysatoren Anwendung finden.

Gegenstand der Erfindung ist ein Verfahren zur Herstellung von Alkylphenylethern oder Alkylphenylalkylthioethern der allgemeinen Formel 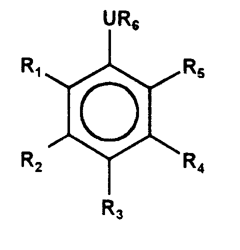
wobei U = O,S,
R₁ bis R₅ gleich oder voneinander verschieden sind und Alkyl- oder Arylgruppen mit 1 bis 6 C-Atomen darstellen, R₆ für eine Alkylgruppe mit 1 bis 6 C-Atomen steht, R₁ bis R₅ auch funktionelle Gruppen wie -COOR, -NO₂, -NH₂, -O-CH₂-CH₂-OH, -OH, -CHO, -H, -Hal sein können, die Reste R₁ bis R₅ aber auch durch bifunktionelle Substituenten, insbesondere -(CH₂)ₓ-, -(CH₂)ₓ-Z-(CH₂)_{y}-, worin Z = Hetero, x = 0-7 und y = 0-7 ist, oder durch olefinisch ungesättigte Substituenten oder durch annelierte Ringsysteme überbrückt sein können, durch Umsetzung der entsprechenden Phenole oder Thiophenole mit Dialkylcarbonaten, welches dadurch gekennzeichnet ist, daß bei Temperaturen von 70-300 °C unter erhöhtem Druck oder Normaldruck in Gegenwart von cyclischen Amidinen als Katalysatoren gearbeitet wird.

Die beschriebene Veretherung wird bei Drücken bis 6 bar durchgeführt. Sie kann aber auch bei Drücken oberhalb von 6 bar durchgeführt werden. Letzteres ist aus ökonomischen Gründen weniger vorteilhaft.

Geeignete Veretherungskatalysatoren sind solche Amidine, in denen die Amidin-Gruppierung Bestandteil eines Ringsystems ist: 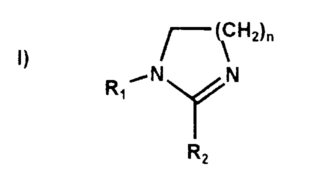
n = 1-6
R₁ =
R₂ = C₁-C₆ Alkyl bzw. Cycloalkyl, Aryl 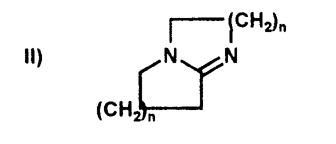
n = 1-6
oder auch 4,5-Dihydro-2-phenylimidazol.

Die Basizität von Amidinen liegt bekanntlich über der von tert.-Aminen, jedoch unterhalb der Basizität von Guanidinen. Es ist deshalb besonders überraschend, daß Amidine besser als Veretherungskatalysatoren geeignet sind, als Guanidine. Gegenüber den tert.-Aminen zeichnen sie sich durch erhöhte Resistenz gegenüber Oxidationsmitteln aus. [R. Schwesinger, Chimia 39, 269 (1985) und Schwesinger, Mißfeldt, Angew. Chemie, 99, 1210 (1987)]. Dies wirkt sich im Vergleich zu Aminen besonders günstig auf die Standfestigkeit des Katalysators aus.

Die Umsetzung von Phenolen mit Dimethylcarbonat kann durch folgende Formel wiedergegeben werden: 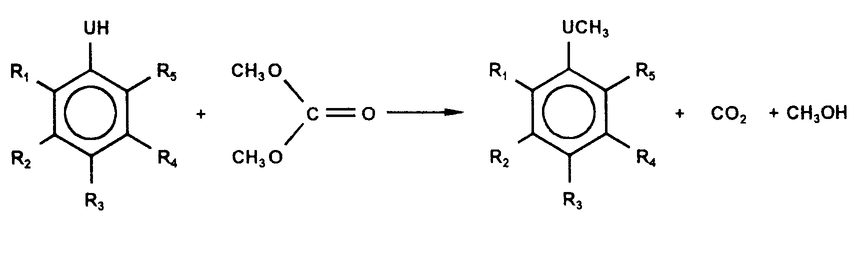
U = O, S

Im Vergleich zu den bekannten Verfahren werden nach der vorliegenden Erfindung Alkylphenylalkylether in sehr guter Ausbeute und hoher Reinheit erhalten. Als Nebenprodukt fallen ausschließlich CO₂ und Alkohol an, die hinsichtlich der Entsorgung als unproblematisch angesehen werden können.

Überraschend war gegenüber den bisher bekannten Verfahren, daß die Reaktion in weniger als 5 Stunden und unter Normaldruck oder unter gering erhöhtem Druck in weniger als 3 Stunden bei den meisten Phenolen zu nahezu vollständigem Umsatz führt (≥ 98 %). Besonders zweckmäßig ist darüber hinaus die häufige Wiederverwendbarkeit des Katalysators, der nach der beendeten Umsetzung nach der Destillation unverändert im Sumpf zurückbleibt. Durch erneute Zugabe von Phenol und Eindosierung von Dimethylcarbonat kann der erfindungsgemäß eingesetzte Katalysator mindestens 10 mal wiederverwendet werden. Dabei wird in allen Fällen nahezu der gleiche Phenolumsatz erzielt wie bei der erstmaligen Verwendung. Das Verfahren kann auf entsprechende Weise auch kontinuierlich betrieben werden.

Es wird sowohl unter Normaldruck als auch unter leicht erhöhtem Druck innerhalb von 2-4 h fast vollständiger Phenolumsatz erzielt, wobei mit oder ohne inerten Lösungsmitteln die gleiche hohe Raum-Zeit-Ausbeute zu verzeichnen ist.

Als Ausgangsphenole kommen beispielsweise in Frage: Phenol, Brenzkatechin, Hydrochinon, Resorzin, Phloroglucin, Pyrogallol, o-, m-, p-Kresol, 2,3-, 2,4-, 2,5-, 2,6-, 3,5-, 3,4-Xylenol, 3-oder 4-Isopropylphenol, 2-, 3-oder 4-n-Butyl-, 2-, 3- oder 4-sec.-Butyl- und 2-, 3- oder 4-Isobutylphenol, 2,4,6-Trimethylphenol, 2-Isopropyl-5-methylphenol, 2-tert.-Butyl-, 4-tert.-Butyl-, 2,5-di-tert.-Butylphenol, 2,5-Dimethyl-4-tert.-butylphenol, 2-Cyclohexylphenol, 4-Cyclohexylphenol, 4-n-Hexylphenol, 4-n-Octylphenol, Salicylsäure(-ester), Salicylaldehyd, Eugenol, Isoeugenol, Vanillin, α- oder β-Naphtol, 2- oder 4-Aminophenol, Brenzkatechinmonomethylester, Guajakol, 4-tert.-Butylbrenzkatechin. In Analogie dazu kommen die entsprechenden Thiophenole ebenfalls in Frage.

Bevorzugte Dialkylcarbonate sind: Dimethylcarbonat, Diethylcarbonat, Methylethylcarbonat, Di-n-propylcarbonat, Di-n-butylcarbonat, Dibenzylcarbonat.

Als Katalysatoren verwendet man Amidine, die zwischen 0,1-10 %, bezogen auf eingesetztes Phenol, vorgelegt werden. Besonders geeignet sind: 1,3-Diazepine, Diazabicyclododecatrien (2,4,6), Diazabicycloundecen, 4,5-Dihydro-2-phenylimidazol.

Es kann auch in Gegenwart von Lösemitteln gearbeitet werden. Geeignete Lösemittel sind inert und hochsiedend, z. B. Xylol, Mesitylen, Nonan, Decan, N-Methylpyrrolidon. Die Temperaturen liegen bei 70-300 °C, vorzugsweise 130 bis 190 °C; angewendet wird Normaldruck oder leicht erhöhter Druck bis 6 bar. Das Verfahren verläuft auch bei Drücken > 6 bar. Es kann kontinuierlich oder diskontinuierlich geführt werden.

Die nach diesem Verfahren synthetisierten Produkte sind wichtige Vorprodukte bzw. schon selbst Riechstoffe, Pharmaka, Pflanzenschutzmittel und Stabilisatoren für Speiseöle.

### Beispiel 1

10 mol p-tert.-Butylphenol werden mit 0,2 mol 1,5-Diazabicyclo-[4.3.0]-non-5-en (DBN) in einen Reaktor mit Rührvorrichtung vorgelegt und zusammen mit einem Lösungsmittel wie Decan auf 160 °C erhitzt. Dazu werden über ein Einleitungsrohr, das bis zum Reaktorboden reicht, 12 mol Dimethylcarbonat im Verlauf von 4 Stunden gleichmäßig eindosiert. Das bei der Reaktion entstehende Methanol wird (zusammen mit dem CO₂) über eine Kolonne abdestilliert. Dadurch ist gewährleistet, daß die Reaktionstemperatur nicht absinkt.

Man erhält nach Beendigung der Reaktion 9,5 mol p-tert.-Butylphenylmethylether, was nach der Destillation 95 % Ausbeute bedeutet. Im Sumpf bleiben 0,18 mol Diazabicyclononen unverändert zurück.

### Beispiel 2

15 mol Eugenol werden mit 0,2 mol 1,8-Diazabicyclo-[5.4.0]-undecen-7 und 16 mol Dimethylcarbonat in einen Druckreaktor vorgelegt, der verschlossen und auf 150 °C erhitzt wird. Nach 2 Stunden kann vollständiger Umsatz von Eugenol festgestellt werden, wobei nach der Destillation 13 mol Eugenolmethylether und 1 mol Isoeugenolmethylether erhalten werden können, was einer Ausbeute von 86 % entspricht.

### Beispiel 3

5 mol β-Naphtol werden mit 6 mol Dimethylcarbonat und 0,25 mol 4,5-Dihydro-2-phenylimidazol in einen Rührautoklav vorgelegt und auf 140 °C erhitzt. Nach 2,5 Stunden kann gaschromatographisch vollständiger Umsatz von β-Naphtol festgestellt werden.

Nach destillativer Aufarbeitung werden 4,5 mol β-Naphtylmethylether erhalten.

### Beispiel 4

Es werden 5 mol o-tert.-Butylphenol mit 400 ml Decan und 5 % 4,5-Dihydro-2-phenylimidazol vorgelegt und auf 140 °C erhitzt. Dazu werden innerhalb von 4 h 5,5 mol Diethylcarbonat zudosiert. Das entstehende Ethanol wird aus dem Reaktionsgemisch kontinuierlich abdestilliert. Nach beendeter Reaktion werden 4,6 mol o-tert.-Butylethoxybenzol aus dem Gemisch durch Destillation erhalten. Die Ausbeute entspricht 92 % der Theorie.

### Beispiel 5

1 mol Thiophenol wird mit 100 ml Nonan und 5 % 1,5-Diazabicyclo-[4.3.0]-non-5-en vorgelegt und auf 150 °C erhitzt. Dazu werden innerhalb von 4 h 1,2 mol Dimethylcarbonat zudosiert. Das entstehende Methanol wird aus dem Reaktionsgemisch abdestilliert. Das Thioanisol wird nach beendeter Reaktion mit einer destillativen Ausbeute von 88 % erhalten.

## Patentansprüche

1. Verfahren zur Herstellung von Alkylphenylalkylethern oder Alkylphenylalkylthioethern der allgemeinen Formel 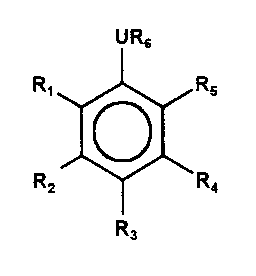
wobei U = O,S,
R₁ bis R₅ gleich oder voneinander verschieden sind und Alkyl- oder Arylgruppen mit 1 bis 6 C-Atomen darstellen, R₆ für eine Alkylgruppe mit 1 bis 6 C-Atomen steht, R₁ bis R₅ auch funktionelle Gruppen wie -COOR, -NO₂, -NH₂, -O-CH₂-CH₂-OH, -OH, -CHO, -H, -Hal sein können, die Reste R₁ bis R₅ aber auch durch bifunktionelle Substituenten -(CH₂)ₓ-, -(CH₂)ₓ-Z-(CH₂)_{y}-, worin Z = Hetero, x = 0-7 und y = 0-7 ist, oder durch olefinisch ungesättigte Substituenten oder durch annelierte Ringsysteme überbrückt sein können, durch Umsetzung der entsprechenden Phenole oder Thiophenole mit Dialkylcarbonaten,
dadurch gekennzeichnet,
daß bei Temperaturen von 70-300 °C und einem Druck von 1 bis 6 bar in Gegenwart von cyclischen Amidinen der allgemeinen Formel 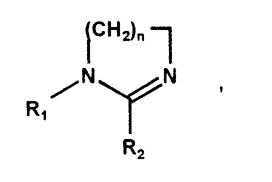
worin n= 1-6 und
R₁ =
R₂ = C₁-C₆-Alkyl, Aryl, Cycloalkyl
bedeuten, oder von cyclischen Amidinen der allgemeinen Formel 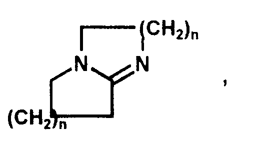
worin n = 1-6 bedeutet,
oder auch von 4,5-Dihydro-2-phenylimidazol als Katalysatoren gearbeitet wird.

2. Verfahren nach Anspruch 1,
dadurch gekennzeichnet,
daß als Katalysator Diazabicycloundecen eingesetzt wird.

3. Verfahren nach den Ansprüchen 1 und 2,
dadurch gekennzeichnet,
daß bei einer Temperatur von 130-190 °C gearbeitet wird.

4. Verfahren nach den Ansprüchen 1 bis 3,
dadurch gekennzeichnet,
daß in Gegenwart eines Lösemittels gearbeitet wird.

5. Verfahren nach den Ansprüchen 1 bis 4,
dadurch gekennzeichnet,
daß das Verfahren auch kontinuierlich und diskontinuierlich betrieben werden kann.

## Claims

1. A process for preparing alkylphenyl alkyl ethers or alkylphenyl alkyl thioethers of the general formula 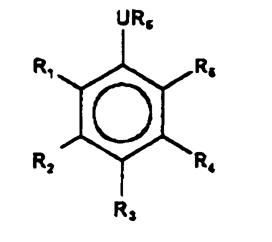
where U = O, S,
R₁ to R₅ are identical to or different from one another and are alkyl or aryl groups having from 1 to 6 carbon atoms, R₆ is an alkyl group having from 1 to 6 carbon atoms, R₁ to R₅ can also be functional groups such as -COOR, -NO₂, -NH₂, -O-CH₂-CH₂-OH, -OH, -CHO, -H and halogen, and the radicals R₁ to R₅ can also be bridged by bifunctional substituents -(CH₂)ₓ-, -CH₂)ₓ-Z-(CH₂)_{y}-, where Z = a heteroatom, x = 0-7 and y = 0-7, or by olefinically unsaturated substituents or by fused-on ring systems, by reacting the corresponding phenols or thiophenols with dialkyl carbonates, characterized in that the reaction is carried out at temperatures of 70-300°C and a pressure of from 1 to 6 bar in the presence of cyclic amidines of the general formula 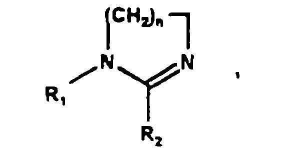
where n = 1-6 and
R₁ =
R₂ = C₁-C₆-alkyl, aryl, cycloalkyl,
of cyclic amidines of the general formula 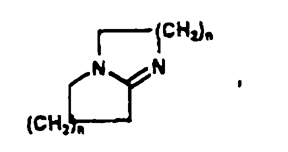
where n = 1-6,
or of 4,5-dihydro-2-phenylimidazole
as catalysts.

2. A process according to claim 1, characterized in that diazabicycloundecene is used as catalyst.

3. A process as claimed in either of claims 1 and 2, characterized in that the reaction is carried out at a temperature of 130-190°C.

4. A process as claimed in any of claims 1 to 3, characterized in that the reaction is carried out in the presence of a solvent.

5. A process as claimed in any of claims 1 to 4, characterized in that the process can be carried out continuously or batchwise.

## Revendications

1. Procédé de préparation d'éthers alkylphénylalkyliques ou de thioéthers alkylphénylalkyliques de formule générale : 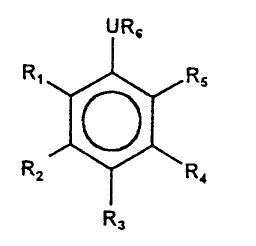
dans laquelle U = 0, S
R₁ à R₅ sont identiques ou différents les uns des autres, et représentent des radicaux alkyle ou aryle ayant de 1 à 6 atomes de carbone,
R₆ représente un radical alkyle ayant de 1 à 6 atomes de carbone,
R₁ à R₅ peuvent être aussi des groupes fonctionnels comme - COOR, -NO₂, -NH₂, -O-CH₂-CH₂-OH,-OH, -CHO, -H, -Hal, les radicaux R₁ à R₅ cependant aussi peuvent être pontés par des substituants bifonctionnels -(CH₂)ₓ-,-(CH₂)ₓ-Z-(CH₂)_{y}-dans lesquels Z = hétéro, x = 0-7 et y = 0-7 ou par des substituants oléfiniquement non saturés ou par des systèmes cycliques annelés
par réaction des phénols ou des thiophénols correspondants, avec des carbonates de dialkyle,
caractérisé en ce qu'
on opère à des températures de 70 à 300°C et à une pression de 1 à 6 bars en présence d'amidines cycliques de formule générale : 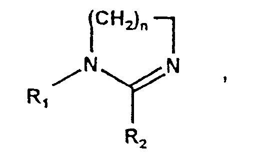
dans laquelle n = 1-6 et,
R1 et R2 signifient un alkyle en C1-C6, un aryle, un cycloalkyle,
d'amidines cycliques de formule générale : 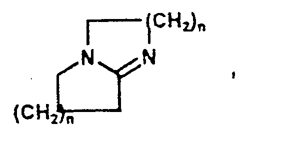
dans laquelle n signifie 1 à 6
ou en présence aussi de 4,5-dihydro-2-phénylimidazole comme catalyseurs.

2. Procédé selon la revendication 1,
caractérisé en ce qu'
on met en oeuvre comme catalyseur le diazabicycloundecène.

3. Procédé selon les revendications 1 et 2,
caractérisé en ce qu'
on opère à une température de 130-190°C.

4. Procédé selon les revendications 1 à 3,
caractérisé en ce qu'
on opère en présence d'un agent dissolvant.

5. Procédé selon les revendications 1 à 4,
caractérisé en ce qu'
on peut conduire le procédé aussi bien en continu que d'une manière discontinue.
